# FASCICULE DE BREVET EUROPEEN

(11) **EP 0 866 418 B1**
(45) Date de publication et mention de la délivrance du brevet: **09.07.2003**
(21) Numéro de dépôt: 98400657.7
(22) Date de dépôt: 20.03.1998
(51) Int. Cl.: G06K 9/00

(54) **Lecteur d'empreintes digitales sécurisé**
Sicherer Fingerabdruckleser
Secure fingerprint reader

(30) Priorité: 21.03.1997 FR 9703482
(43) Date de publication de la demande: 23.09.1998
(73) Titulaire: SAGEM SA, 75116 Paris (FR)
(72) Inventeur: Euverte, Jean-Michel, 95490 Vaureal (FR); Hallibert, Pascal, 75013 Paris (FR)
(74) Mandataire: Bloch, Gérard

(56) Documents cités:
- EP-A- 0 194 783
- EP-A- 0 359 554
- EP-A- 0 640 933
- US-A- 4 977 601
- SCHNEIDER J K ET AL: "LIVE SCAN FINGERPRINT IMAGERY USING HIGH RESOLUTION C-SCAN ULTRASONOGRAPHY" PROCEEDINGS OF THE ANNUAL INTERNATIONAL CARNAHAN CONFERENCE ON SECURITY TECHNOLOGY, TAIPEI, OCT. 1 - 3, 1991, no. CONF. 25, 1 octobre 1991, INSTITUTE OF ELECTRICAL AND ELECTRONICS ENGINEERS, pages 88-95, XP000300413
- FAINZILBERG L ET AL: "Computer analysis and recognition of cognitive phase space electro-cardio graphic image" COMPUTER ANALYSIS OF IMAGES AND PATTERNS. 6TH INTERNATIONAL CONFERENCE, CAIP'95. PROCEEDINGS, PROCEEDINGS OF 6TH INTERNATIONAL CONFERENCE ON COMPUTER ANALYSIS OF IMAGES AND PATTERNS, PRAGUE, CZECH REPUBLIC, 6-8 SEPT. 1995, ISBN 3-540-60268-2, 1995, BERLIN, GERMANY, SPRINGER-VERLAG, GERMANY, pages 668-673, XP002045310

## Description

Un lecteur d'empreinte digitale, en fait des sillons à la surface d'un doigt formant des creux et des bosses qu'on appellera donc par la suite empreinte digitale, sert à identifier une personne par comparaison de son empreinte digitale aux empreintes d'une bibliothèque préalablement constituée.

Il est cependant possible de leurrer le lecteur, car il reconnaît une personne à travers son doigt, et on peut donc lui présenter par exemple un moulage du doigt d'une personne dont il a les empreintes en bibliothèque, ou même encore un doigt coupé de cette personne, bref l'empreinte d'un corps sans vie.

Comme le lecteur sert en général à contrôler l'accès à des informations confidentielles ou à des biens précieux, il importe donc de se prémunir contre ce genre de piratage.

La présente invention vise à augmenter la résistance du lecteur à l'égard des tentatives frauduleuses évoquées ci-dessus.

On connaît déjà, par US 4977601, un lecteur d'empreinte par ultrasons. Par EP-A.0359554, on connaît un lecteur d'empreinte avec des moyens de détection de l'authenticité du doigt par les caractéristiques des réflexions de lumière.

La présente demande propose d'améliorer encore la vérification de l'authenticité dans le cadre d'une lecture optique et de mieux lier la vérification et la lecture.

Ainsi, l'invention concerne un lecteur selon la revendication 1.

Un doigt réel, authentique, va en effet modifier d'une certaine façon une onde ultrasonore qui l'atteint et cette modification est une signature permettant d'authentifier un doigt et d'écarter un leurre, l'identification en elle-même étant effectuée par les empreintes.

Avantageusement, il est prévu des moyens émetteurs et des moyens récepteurs de lumière, à plusieurs longueurs d'onde, agencés pour respectivement illuminer le doigt et fournir un spectre de couleur de la lumière réfléchie par celui-ci à des moyens de comparaison agencés pour authentifier le doigt par comparaison de son spectre de colorimétrie à des spectres de référence, ou encore il est prévu des moyens d'émission de lumière et des moyens de réception de lumière associés à des moyens de traitement de signal, agencés pour effectuer une suite de mesures de la lumière réfléchie par le doigt et en détecter par photoplétysmographie des pulsations cardiaques.

On peut ainsi, en particulier, dans chaque cas, détecter qu'il s'agit d'un doigt vivant.

L'invention sera mieux comprise à l'aide de la description suivante d'un lecteur d'empreinte digitale selon l'invention, en référence aux dessins annexés, sur lesquels :
- la figure 1 représente schématiquement un ensemble à ultrasons, d'analyse des échos d'un doigt,
- la figure 2 illustre les mesures des échos ci-dessus,
- la figure 3 représente schématiquement un ensemble de mesure du spectre colorimétrique d'un doigt,
- la figure 4 illustre le résultat des mesures ci-dessus,
- la figure 5 représente un ensemble de détection par photoplétysmographie des pulsations cardiaques perçues dans le doigt et
- la figure 6 illustre très schématiquement le fonctionnement d'ensemble du lecteur.

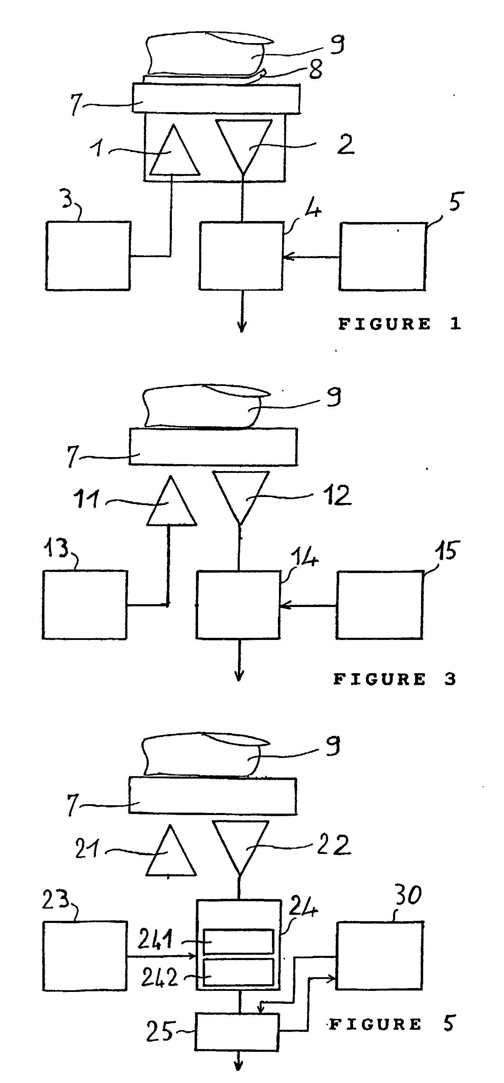

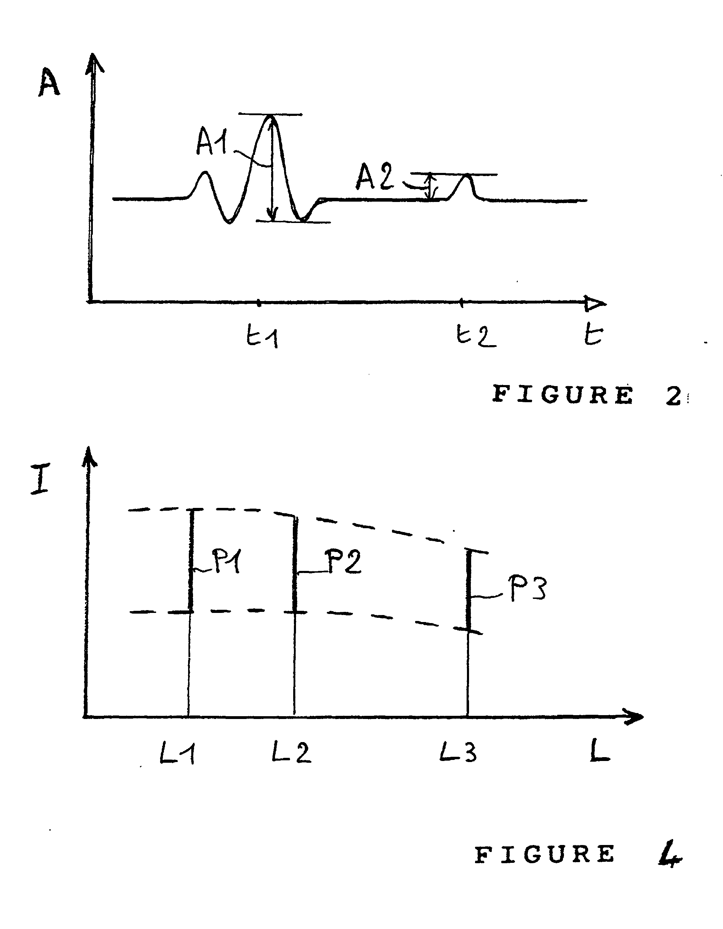

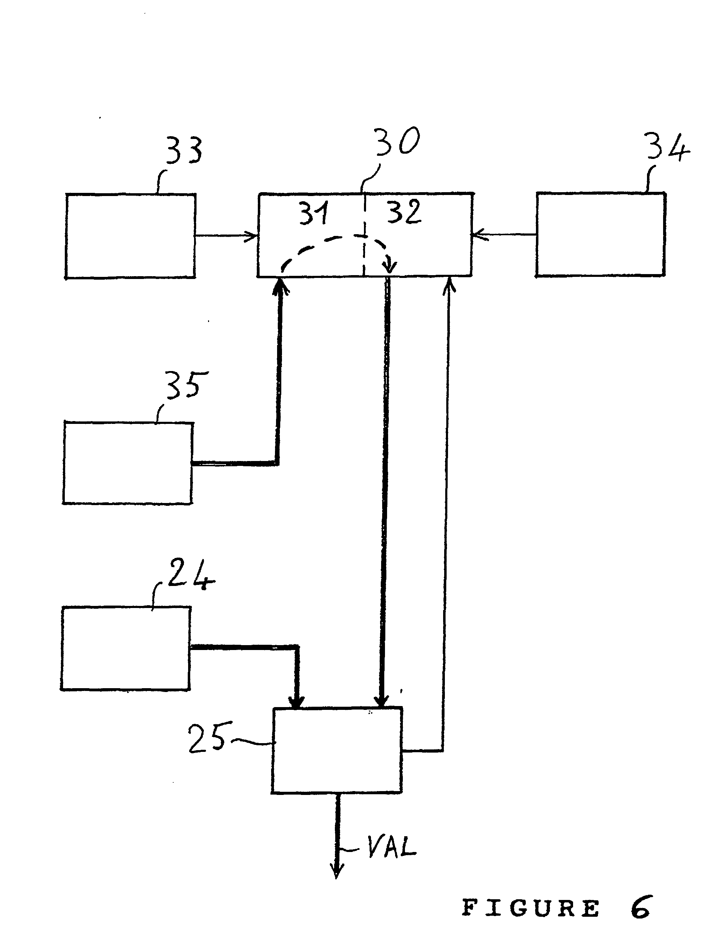

Sur la figure 1 est représenté schématiquement un ensemble à ultrasons, comportant une cellule comprenant un émetteur d'ultrasons 1 et un récepteur associé 2. Une base de temps 3 assure le séquencement des divers circuits et en particulier commande l'émetteur 1, tandis que le récepteur 2 est relié à une entrée d'un comparateur 4 dont une autre entrée est reliée à une mémoire 5 contenant des spectres d'échos de doigts préalablement authentifiés et associés chacun à une identité de personne. La sortie du comparateur 4 fournit, ou non, un signal d'authentification d'un doigt 9 à présenter au lecteur et posé sur une face de lecture d'un bloc optique 7 servant, de façon classique, à illuminer le doigt 9 et à en saisir l'image des reliefs, ou empreinte, par des moyens non représentés. La cellule 1, 2 est accolée à la face du bloc optique 7 opposée à celle de lecture. Dans cet exemple, il s'agit du doigt d'une personne non reconnue par le lecteur, doigt qui est recouvert d'une couche 8 d'un matériau portant l'empreinte d'un doigt reconnaissable, c'est-à-dire mémorisée dans une bibliothèque 31 (fig. 6) d'empreintes du lecteur.

La figure 2 illustre la forme de l'écho obtenu en sortie de récepteur 2, l'amplitude A de l'écho étant portée en ordonnée et le temps t en abscisse.

L'écho présente deux zones de perturbations autour des instants t1 et t2. A l'instant t1, il s'agit de la détection de la peau du doigt 9 présenté, au contact de la couche 8, tandis qu'à l'instant t2 il s'agit de la surface opposée de la couche 8, en contact avec le bloc. La présence de l'impulsion, d'amplitude A2, à l'instant t2 permet la détection de la couche 8.

En outre, l'amplitude A1 des perturbations à l'instant t1 peut être inférieure à l'amplitude de A0 homologue qui serait observée en l'absence de couche 8, si bien que la comparaison entre les perturbations (A1) mesurées à l'instant t1 et celles mémorisées (A0) en l'absence de couche 8 permet de déterminer une atténuation (A0-A1) indiquant, si elle dépasse un seuil, la présence de la couche 8. La comparaison des perturbations peut être effectuée dans le domaine temporel ou dans le domaine fréquentiel. Hormis l'amplitude, la durée des perburbations A1 permet d'identifier un écho de doigt.

La détection de la présence de la couche 8 intervient, au niveau du comparateur 4, par comparaison de la forme d'onde de l'écho ci-dessus à une forme d'onde de référence. Cette référence est mémorisée dans la mémoire 5 et comporte une seule perturbation, qui correspond à la perturbation de l'instant t1, représentée, mais qui n'aurait pas été déformée par les traversées aller-retour de la couche 8.

La déformation ainsi détectée inhibe toute authentification issue du comparateur 4. En outre, la présence d'un second écho permet, indépendamment, d'effectuer la détection de la couche 8, ou simplement de confirmer la détection précédente effectuée par examen de la forme de l'écho, ou spectre amplitude/fréquence, à l'instant t1 prévu.

Sur la figure 3 est représenté schématiquement un ensemble de mesure du spectre colorimétrique du doigt 9 présenté et d'authentification de celui-ci.

La structure représentée ne sera pas décrite en détails car elle est quasiment semblable à celle de la figure 1, les émetteur et récepteur étant ici optiques. Les éléments homologues de ceux de la figure 1 portent la même référence d'unité précédée de la dizaine 1, le bloc 7 étant inchangé.

L'émetteur 11 est à plusieurs longueurs d'onde, ici de la lumière blanche, et le récepteur 12 détecte plusieurs longueurs d'onde, ici trois.

La figure 4 illustre le résultat des mesures, c'est-à-dire le spectre de l'intensité optique détectée I en fonction de la longueur d'onde L .

Pour chaque longueur d'onde L1, L2, L3, la mémoire 15 comporte une plage P1, P2, P3 d'intensités acceptables, définissant le spectre colorimétrique du doigt 9 et ses variations acceptables. Les deux lignes en pointillés délimitent une pluralité de plages, ou spectre, pour une bande de fréquences. Un doigt est alors authentifié si les trois mesures d'intensité sont dans la plage prévue pour les trois longueurs d'onde respectives.

La figure 5 représente schématiquement un ensemble de détection des pulsations cardiaques perceptibles dans un doigt vivant par photoplétysmographie, c'est-à-dire détection des augmentations de volume des vaisseaux sanguins dues aux augmentations de pression liées au battement cardiaque.

Cet ensemble comporte un émetteur de lumière 21 et un récepteur de lumière associé 22 disposés contre la face du bloc 7 opposée à celle recevant le doigt 9. Le récepteur 22 alimente un circuit 24 de traitement du signal qui détermine, par une suite mesures, l'amplitude et la fréquence des variations du signal optique renvoyé par le doigt 9.

A cet effet, un circuit 241 de mesure d'amplitude détermine la valeur crête d'impulsions cardiaques par rapport à la valeur de repos séparant ces impulsions. De même, un circuit fréquencemètre ou périodemètre 242 mesure la période de répétition de ces impulsions pour déterminer la fréquence cardiaque correspondante. Une base de temps 23 fournit les signaux d'horloge nécessaires.

Ainsi, par photoplétysmographie, on vérifie que le doigt 9 est vivant et on détermine les caractéristiques cardiaques de la personne contrôlée : rythme des pulsations et amplitude.

Une bibliothèque 30 contient un historique comportant, pour chaque personne autorisée, son empreinte associée à ses caractéristiques photoplétysmographiques. Cette bibliothèque 30 est adressée (figure 6) par l'ensemble classique 35 de reconnaissance d'empreinte et fournit l'identité de la personne contrôlée, si elle est ainsi reconnue. De plus, la bibliothèque 30 fournit, à un circuit comparateur 25 alimenté par le circuit de traitement 24, les caractéristiques photoplétysmographiques associées à l'empreinte. Ici, il est prévu de fournir des plages de tolérance de ces caractéristiques. Le circuit 25 compare alors les caractéristiques mesurées à celles fournies par la bibliothèque 30 qu'il consulte pour authentifier ou non la personne contrôlée. Il aurait pu être prévu que ce soit le circuit de traitement 24 qui adresse la bibliothèque 30 pour en sélectionner un groupe d'identités de personnes, dans lequel l'ensemble de reconnaissance d'empreintes 35 doit rechercher l'empreinte de la personne contrôlée.

Dans cet exemple, les plages de tolérance des caractéristiques sont définies de façon adaptative, c'est-à-dire que le circuit 25 fournit en retour à la bibliothèque 30 les valeurs des caractéristiques mesurées, s'il les a validées, afin de constituer, pour chaque personne, un historique de ses caractéristiques. Partant de plages de tolérance initiales, à la mise en service, relativement étendues, l'historique ou histogramme, permet de réduire et ainsi personnaliser progressivement ces plages à mesure qu'il s'enrichit et améliore ainsi l'efficacité du contrôle.

Afin de limiter le volume de matériel, il est ici en fait prévu une source lumineuse et un capteur optique, multifonctionnels, de colorimétrie et aussi de photoplétysmographie. Dans ce même but, la source lumineuse et le capteur appartiennent à l'ensemble de reconnaissance d'empreintes 35.

La figure 6 illustre le fonctionnement global du lecteur. Par souci de clarté, les ensembles de mesure des figures 1 et 3 n'y ont pas été représentés. La bibliothèque 30 est constituée de deux sous-ensembles 31 et 32, respectivement d'empreintes et des caractéristiques biologiques associées de chaque personne autorisée.

Pour constituer initialement la bibliothèque 30, l'ensemble 35 lit plusieurs fois l'empreinte digitale des diverses personnes autorisées, sous la commande d'un circuit 33 y associant leur identité. Une base de données 34, comportant les caractéristiques biologiques de l'espèce humaine, sert à alimenter le sous-ensemble 32.

En exploitation, l'ensemble 35 fournit une empreinte de doigt présentée au sous-ensemble 31 et ce dernier fournit, le cas échéant, l'identité de la personne reconnue. Cette identité permet d'accéder aux caractéristiques biologiques (32) de cette personne, caractéristiques qui sont initialement identiques pour toutes les personnes mais qui sont personnalisées par la suite, comme cela a été expliqué précédemment.

Le sous-ensemble 32 fournit alors ces caractéristiques biologiques au circuit comparateur 25 alimenté aussi par le circuit de lecture 24 et valide (VAL) ou non la reconnaissance de l'identité de la personne, préalablement reconnue par son empreinte. Comme indiqué, le circuit 25 fournit en retour, au sous-ensemble 32, la caractéristique mesurée et acceptée, pour constituer l'historique. L'absence de toute réponse du sous-ensemble 31 invalide donc le circuit 25.

On conçoit que chacun des trois ensembles de mesure des figures 1, 3 et 5 suffit en lui-même pour authentifier un doigt en tant que tel et/ou pré-sélectionner une personne parmi plusieurs, et ainsi atteindre le but visé. La combinaison de plusieurs de ces ensembles a pour effet d'accroître l'efficacité de filtrage entre personnes, donc affecte son degré et non la nature du résultat.

## Revendications

1. Lecteur optique d'empreinte digitale, dans lequel il est prévu un émetteur d'ultrasons (1), agencé pour émettre des ultrasons vers un doigt (9) à présenter au lecteur, un récepteur d'ultrasons (2), disposé pour recevoir les ultrasons issus du doigt (9), et des moyens comparateurs (4), reliés au récepteur (2) et à une mémoire (5) de spectres d'échos de doigts préalablement authentifiés et agencés pour comparer les spectres amplitude/fréquence des ultrasons émis et reçus et les spectres de la mémoire (5), et ainsi vérifier qu'il s'agit d'un doigt (9) authentique, et, pour détecter l'écho d'une couche inerte (8) recouvrant un doigt (9).

2. Lecteur selon la revendication 1, dans lequel il est prévu des moyens émetteurs (11) et des moyens récepteurs (12) de lumière, à plusieurs longueurs d'onde, agencés pour respectivement illuminer le doigt (9) et fournir un spectre de couleur de la lumière réfléchie par celui-ci à des moyens de comparaison (14) agencés pour authentifier le doigt (9) par comparaison de son spectre de colorimétrie à des spectres de référence (15).

3. Lecteur selon l'une des revendications 1 et 2, dans lequel il est prévu des moyens d'émission de lumière (21) et des moyens de réception de lumière (22) associés à des moyens de traitement de signal (24), agencés pour effectuer une suite de mesures de la lumière réfléchie par le doigt (9) et en détecter par photoplétysmographie des pulsations cardiaques.

4. Lecteur selon la revendication 3, dans lequel les moyens de traitement (24) comportent des moyens de mesure de fréquence (241) agencés pour déterminer une caractéristique de rythme des pulsations d'un doigt (9).

5. Lecteur selon l'une des revendications 3 et 4, dans lequel les moyens de traitement comportent des moyens de mesure d'amplitude (242) agencés pour déterminer une caractéristique d'amplitude des pulsations d'un doigt (9).

6. Lecteur selon l'une des revendications 4 et 5, dans lequel les moyens de traitement (24, 25) sont agencés pour consulter un historique (30) d'au moins l'une des caractéristiques d'un doigt (9) et pour authentifier ce doigt par comparaison entre la mesure et l'historique.

7. Lecteur selon la revendication 6, dans lequel les moyens de traitement (24, 25) sont agencés pour constituer l'historique (30).

8. Lecteur selon l'une des revendications 2 à 7, dans lequel il est prévu une source lumineuse et un capteur optique communs de colorimétrie et de photoplétysmographie.

9. Lecteur selon la revendication 8 dans lequel la source lumineuse et le capteur appartiennent à un ensemble de reconnaissance d'empreintes.

## Patentansprüche

1. Optischer Fingerdruckableser, in welchem ein Ultraschallsender (1) vorgesehen ist, der angelegt ist, um einem dem Leser vorzustellenden Finger (9) Ultraschall zuzusenden, ein Ultraschallempfänger (2), der angeordnet ist, um den vom Finger (9) ausgehenden Ultraschall zu empfangen und Vergleichsmittel (4), die mit dem Empfänger (2) und einem Echospektrumsspeicher (5) von vorangehend authentifizierten Fingern verbunden sind und angelegt sind, um die Frequenz-/Amplitudenspektren des gesandten und empfangenen Ultraschalls mit den Spektren des Speichers (5) zu vergleichen und somit zu prüfen, ob es sich um einen echten Finger (9) handelt, und um das Echo einer leblosen, den Finger (9) überdeckenden Schicht (8) festzustellen.

2. Leser nach Anspruch 1, in welchem Lichtsendemittel (11) und Lichtempfangsmittel (12) mit verschiedenen Wellenlängen vorgesehen sind, die angelegt sind, um den Finger (9) jeweils zu beleuchten und ein Farbspektrum des von ihm reflektierten Lichtes an Vergleichsmittel (14) zu liefern, um den Finger (9) durch den Vergleich zwischen seinem Farbmessungsspektrum und Referenzspektren (15) zu authentifizieren.

3. Leser nach einem der Ansprüche 1 und 2, in dem Lichtsendemittel (21) und Lichtempfangsmittel (22) vorgesehen sind, die Signalverarbeitungsmitteln (24) zugeordnet sind, welche angelegt sind, um eine Reihe von Messungen des vom Finger (9) reflektierten Lichtes vorzunehmen und um dadurch und durch Photopletysmographie das Herzklopfen festzustellen.

4. Leser nach Anspruch 3, in welchem die Verarbeitungsmittel (24) Frequenzmessmittel (241) aufweisen, die angelegt sind, um eine rhythmische Eigenschaft der Pulsierung eines Fingers (9) festzustellen.

5. Leser nach einem der Ansprüche 3 und 4, in welchem die Verarbeitungsmittel Amplitudenmessmittel (242) aufweisen, die angelegt sind, um eine Eigenschaft der Amplitude der Pulsierung eines Fingers (9) zu bestimmen.

6. Leser nach einem der Ansprüche 4 und 5, in welchem die Verarbeitungsmittel (24, 25) angelegt sind, um eine geschichtliche Aufzeichnung (30) mindestens einer der Eigenschaften eines Fingers (9) abzufragen und um diesen Finger durch den Vergleich zwischen der Messung und der geschichtlichen Aufzeichnung zu authentifizieren.

7. Leser nach dem Anspruch 6, in welchem die Verarbeitungsmittel (24, 25) angelegt sind, um die geschichtliche Aufzeichnung (30) aufzubauen.

8. Leser nach einem der Ansprüche 2 bis 7, in welchem eine Lichtquelle und ein optischer Sensor gemeinsam für die Farbmessung und die Photopletysmographie vorgesehen sind.

9. Anspruch nach Anspruch 8, in welchem die Lichtquelle und der Sensor zu einer Abdruckserkennungsbaugruppe gehören.

## Claims

1. An optical fingerprint reader, wherein it is provided with an ultrasound emitter (1) configured for transmitting ultrasound to a finger (9) for presentation to a reader, an ultrasound receiver (2), arranged for receiving the ultrasound sent from the finger (9), and comparator means (4) connected to a receiver (2) and to a memory (5) for echo spectra of fingers previously authenticated and configured for comparing the amplitude / frequency spectra of the ultrasound transmitted and received and the spectra of the memory (5) and in this fashion to verify that the finger (9) is authentic and for detecting the echo of an inert layer (8) covering a finger (9).

2. The reader according to Claim 1, wherein it is provided with light emitting means (11) and light receiving means (12) at a plurality of wavelengths, configured respectively for illuminating the finger (9) and providing a color spectrum of the light reflected by the latter to comparison means (14) configured for authenticating the finger (9) by comparison of its colorimetric spectrum to reference spectra (15).

3. The reader according to Claims 1 and 2, wherein it is provided with light emitting means (21) and light receiving means (22) associated with signal processing means (24), configured for carrying out a series of measurements of the light reflected by the finger (9) and detecting thereby cardiac pulsations by using photoplethysmography.

4. The reader according to Claim 3, wherein the processing means (24) comprise frequency measuring means (241) configured for determining a rhythm characteristic of the pulsations of a finger (9).

5. The reader according to one of Claims 3 and 4, wherein the processing means comprise amplitude measuring means (242) configured for determining an amplitude characteristic of the pulsations of a finger (9).

6. The reader according to one of Claims 4 and 5, wherein the processing means (24, 25) are configured for consulting a record (30) of at least one of the characteristics of a finger (9) and for authenticating said finger by comparison between the measurement and the record.

7. The reader according to Claim 6, wherein the processing means (24, 25) are configured for constituting the record (30).

8. The reader according to one of Claims 2 to 7, wherein it is provided with a common light source and a common optical sensor for colorimetry and photoplethysmography.

9. The reader according to Claim 8, wherein the light source and the sensor are part of an assembly for recognizing prints.
